# EUROPEAN PATENT APPLICATION

(11) **EP 4 563 068 A1**
(43) Date of publication of application: **04.06.2025**
(21) Application number: 23846021.6
(22) Date of filing: 06.06.2023
(51) Int. Cl.: A61B 1/12, G02B 23/24, H05K 7/20

(54) **ELECTRONIC ENDOSCOPE PROCESSOR**

(30) Priority: 28.07.2022 JP 2022120374
(71) Applicant: HOYA CORPORATION, Shinjuku-ku Tokyo 160-8347 (JP)
(72) Inventor: HIJIKATA, Takao, Tokyo 160-8347 (JP)
(74) Representative: Schaumburg und Partner Patentanwälte mbB
(86) International application number: PCT/JP2023/021007
(87) International publication number: WO 2024/024285

(57) **Abstract**

One aspect of the present invention is an electronic endoscope processor including: a housing; a partition plate that partitions an internal space of the housing into a first space below and a second space above, the partition plate having a first aperture that is an aperture allowing the first space and the second space to communicate with each other; and an exhaust fan that is attached to the housing and exhausts air in the first space to the outside. A first intake hole through which outside air is acquired into the first space and a second intake hole through which outside air is acquired into the second space are formed in the housing.

## Description

### Technical Field

The present invention relates to an electronic endoscope processor configured to acquire and process a captured image of a living tissue.

### Background Art

An electronic endoscope system is used to observe or treat a living tissue inside a human body. The electronic endoscope system includes an electronic endoscope that captures an image of a living tissue with an image sensor and transmits the captured image to a processor, and the processor (electronic endoscope processor) that processes a signal of the captured image to create an image for display.

In the electronic endoscope processor, a power supply circuit and a circuit board including an integrated circuit such as a CPU or a field-programmable gate array (FPGA) in order to secure high performance processing capability are used, and further, a light source device for observing a living tissue may be built in the processor. Since such a heat source is accommodated in a housing of the processor, various measures have been conventionally proposed in order to mitigate a temperature rise inside the processor due to a long-time operation.

For example, JP 2019-107291 A proposes a processor including: a heat insulating plate that vertically partitions a space near an exhaust port in a housing to form a first internal space on a lower side and a second internal space on an upper side; and a single cooling fan that exhausts air that has flowed into the first internal space and the second internal space from an intake port to the outside. This processor includes an exhaust mechanism that exhausts heat inside both the first internal space on the lower side and the second internal space on the upper side by the single cooling fan.

### Summary of Invention

### Technical Problem

However, in the exhaust mechanism described in JP 2019-107291 A, both air in the first internal space on the lower side and air in the second internal space on the upper side are simultaneously exhausted to the outside by one fan, and thus, there is a problem that an air flow sucked by the fan from each of the first internal space and the second internal space becomes non-uniform. Therefore, there is a possibility that cooling performance varies when there is a heat source in each of the first internal space and the second internal space.

Therefore, an object of the present invention is to provide an electronic endoscope processor capable of cooling an internal space of a housing in a well-balanced manner.

### Solution to Problem

One aspect of the present invention is an electronic endoscope processor including:
a housing;
a partition plate that partitions an internal space of the housing into a first space below and a second space above, the partition plate having a first aperture that is an aperture allowing the first space and the second space to communicate with each other; and
an exhaust fan that is attached to the housing and exhausts air in the first space to the outside.

A first intake hole through which outside air is acquired into the first space and a second intake hole through which outside air is acquired into the second space are formed in the housing.

The electronic endoscope processor includes one or a plurality of heat generating components that are arranged in the first space and generate heat during operation. In this case, the electronic endoscope processor preferably includes an air flow adjusting plate that is arranged between the first aperture and each of the heat generating components in the first space and adjusts an air flow entering the first space from the first aperture to be directed to each of the heat generating components.

A second aperture that is an aperture for adjusting a wind speed and/or a wind amount of the air flow directed to each of the heat generating components may be formed in the air flow adjusting plate.

The second intake hole is preferably larger than the first intake hole.

A size of the first intake hole is preferably set such that an air flow is the fastest in the first space and the second space.

A peripheral edge of the first aperture may have a turbulence generating structure that generates turbulence in an air flow passing through the first aperture. A peripheral edge of the second aperture may have a turbulence generating structure that generates turbulence in an air flow passing through the second aperture.

### Advantageous Effects of Invention

According to the above-described electronic endoscope processor, the internal space of the housing can be cooled in a well-balanced manner.

### Brief Description of Drawings

Fig. 1 is a block diagram illustrating an overall configuration of an electronic endoscope system of an embodiment.
Fig. 2 is a perspective view and a rear view of the electronic endoscope processor according to the embodiment.
Fig. 3 is a schematic exploded perspective view of the electronic endoscope processor according to the embodiment.
Fig. 4 is a view of the inside of the electronic endoscope processor according to the embodiment as viewed from the side.
Fig. 5 is a view of a lower space of a housing of the electronic endoscope processor according to the embodiment as viewed from above.
Fig. 6 is a view of the inside of the electronic endoscope processor according to the embodiment as viewed from the front.
Fig. 7 is a view for describing an air flow in an internal space of the electronic endoscope processor according to the embodiment.
Fig. 8 is a view for describing an air flow when the inside of the electronic endoscope processor according to the embodiment is viewed from the side.
Fig. 9 is a view illustrating a state in which a protrusion is provided in the electronic endoscope processor according to the embodiment.

### Description of Embodiments

### (System Configuration)

Hereinafter, an electronic endoscope system according to an embodiment will be described.

An electronic endoscope system according to the embodiment includes: an electronic endoscope (electronic scope) including an image sensor that acquires a captured image of a living tissue; and an electronic endoscope processor detachably connected to the electronic endoscope. The electronic endoscope processor is an electronic device that performs signal processing on the captured image of the living tissue to create an image for display.

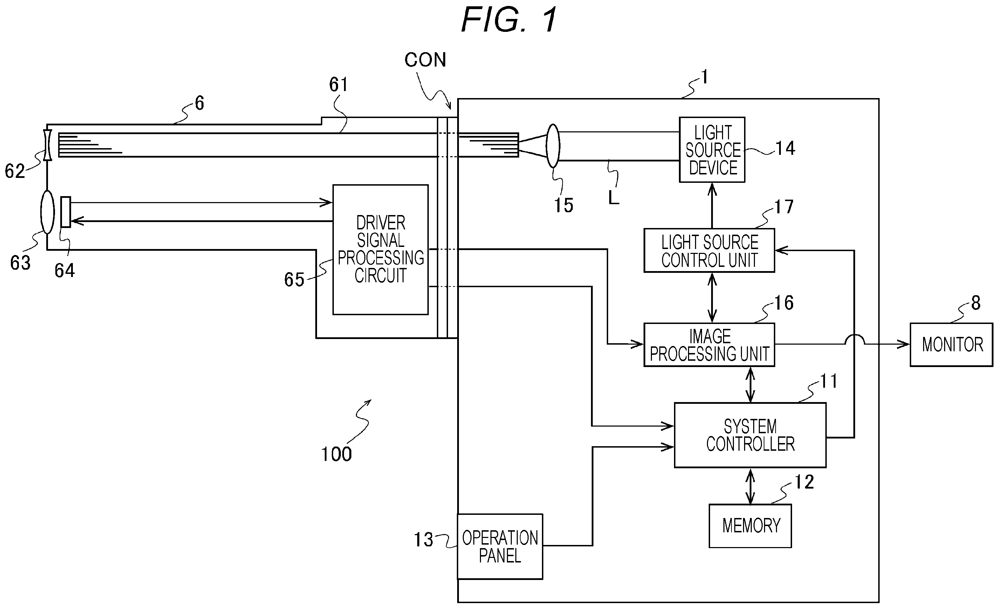

Fig. 1 illustrates a schematic configuration of an electronic endoscope system 100 according to the embodiment. As illustrated in Fig. 1, the electronic endoscope system 100 includes an electronic endoscope processor 1, an electronic scope 6, and a monitor 8. The electronic endoscope processor 1 and the electronic scope 6 are connected by a connector CON.

The electronic endoscope processor 1 includes a system controller 11. The system controller 11 executes various programs stored in a memory 12 and integrally controls the entire electronic endoscope system 100. Further, the system controller 11 is connected to an operation panel 13. The system controller 11 changes operations of the electronic endoscope system 100 and a parameter for each of the operations in accordance with an operator's instruction input to the operation panel 13.

The electronic endoscope processor 1 includes a light source device 14. The light source device 14 emits illumination light L for illuminating an object such as a living tissue in a body cavity. A light source of the light source device 14 is, for example, a high luminance lamp (for example, a xenon lamp, a metal halide lamp, a mercury lamp, a halogen lamp, or the like) that emits white illumination light, a plurality of light emitting diodes emitting light in a wavelength band of a predetermined color, or a laser light source. The illumination light L emitted from the light source device 14 is condensed onto an incident end face of a light carrying bundle (LCB) 61 by a condenser lens 15, and is incident into the LCB 61.

The illumination light L incident into the LCB 61 propagates through the LCB 61. The illumination light L propagating through the LCB 61 is emitted from an exit end face of the LCB 61 arranged at a distal tip of the electronic scope 6, and is applied to the object via a light distribution lens 62. Return light from the object illuminated with the illumination light L from the light distribution lens 62 forms an optical image on a light receiving surface of a solid-state image sensor 64 via an objective lens 63.

The solid-state image sensor 64 is a single-plate color charge coupled device (CCD) image sensor having a Bayer pixel arrangement. The solid-state image sensor 64 accumulates an optical image formed by each of pixels on the light receiving surface, as charge corresponding to the amount of light, and generates and outputs image signals of Red (R), Green (G), and Blue (B). Note that the solid-state image sensor 64 is not limited to a CCD image sensor, and may be a complementary metal oxide semiconductor (CMOS) image sensor.

A driver signal processing circuit 65 is provided in a connection portion of the electronic scope 6. An image signal of the object is input to the driver signal processing circuit 65 from the solid-state image sensor 64 in a predetermined frame cycle. For example, the frame cycle is 1/30 seconds. The driver signal processing circuit 65 performs predetermined processing including A/D conversion on the image signal input from the solid-state image sensor 64 and outputs the processed image signal to an image processing unit 16 of the electronic endoscope processor 1.

The image processing unit 16 performs predetermined image processing to generate a video format signal, and outputs the video format signal to the monitor 8.

The electronic endoscope processor 1 includes a light source control unit 17 that acquires luminance information of the image signal from the image processing unit 16 and controls the intensity of the illumination light of the light source device 14 based on the luminance information.

The light source control unit 17 controls the light source device 14 such that the intensity of the illumination light emitted from the light source device 14 is high when brightness is low and the intensity of the illumination light emitted from the light source device 14 is low when brightness is high. Accordingly, the brightness of the image signal received from the driver signal processing circuit 65 is controlled to be maintained constant.

The system controller 11 performs various calculations based on unique information of the electronic scope 6, and generates a control signal. The system controller 11 controls operations and timings of various circuits in the electronic endoscope processor 1 using the generated control signal such that processing suitable for the electronic scope 6 connected to the electronic endoscope processor 1 is performed.

### (Structure of Electronic Endoscope Processor 1)

Next, a structure of the electronic endoscope processor 1 will be described.

In the electronic endoscope processor 1, each of the system controller 11, the image processing unit 16, and the light source control unit 17 is a heat generating component configured by a circuit board including a central processing unit (CPU), an application specific integrated circuit (ASIC), a field-programmable gate array (FPGA), or the like and generates heat with the operation of the electronic endoscope processor 1. The light source device 14 is a heat generating component that generates heat with light emission of the light source. In addition, heat generating components such as a power supply board and a heat sink are mounted on the electronic endoscope processor 1.

As described above, since the plurality of heat generating components need to be included in the housing constituting the electronic endoscope processor 1, the inside of the housing is designed such that an internal space of the housing can be cooled in a well-balanced manner.

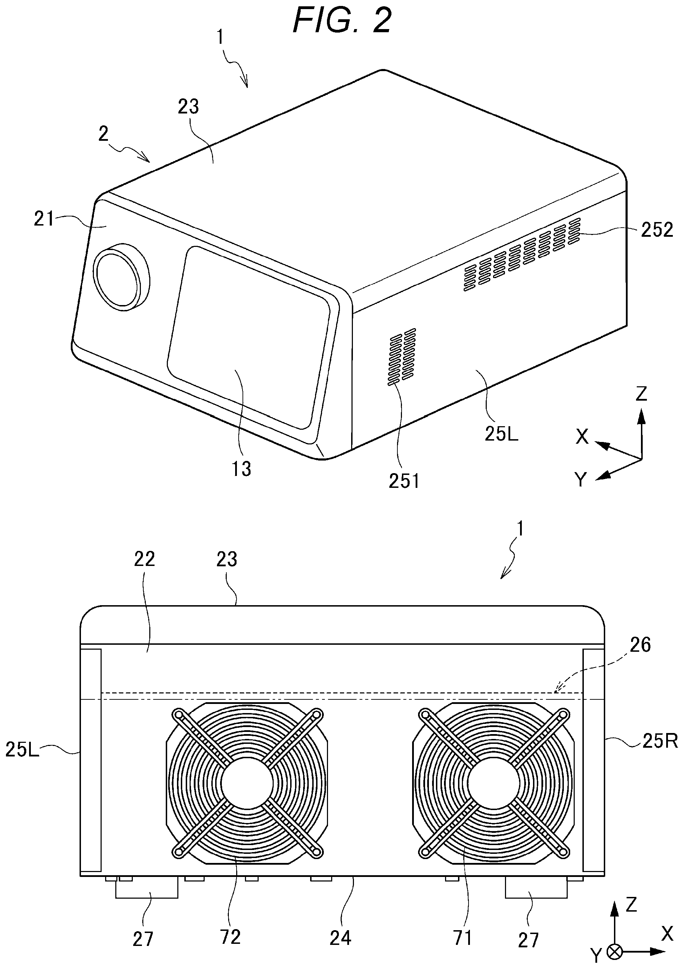

Fig. 2 illustrates a perspective view and a rear view of the electronic endoscope processor 1 according to the embodiment.

As illustrated in Fig. 2, a housing 2 of the electronic endoscope processor 1 includes a front surface portion 21, a rear surface portion 22, a top plate 23, a bottom plate 24, a left side plate 25L, and a right side plate 25R, and has an appearance of a rectangular parallelepiped shape. A plurality of legs 27 are attached to the bottom plate 24.

The operation panel 13 is arranged on the front surface portion 21. The left side plate 25L has an intake hole 251 (an example of a first intake hole) and an intake hole 252 (an example of a second intake hole) through which outside air is introduced into the housing 2. Note that the intake holes 251 and 252 may also be symmetrically formed in the right side plate 25R although not visible in Fig. 2. In the following description, the left side plate 25L and the right side plate 25R are described as a "side plate 25" when matters common to the left side plate 25L and the right side plate 25R are referred to.

Two exhaust fans 71 and 72 are installed on the rear surface portion 22 of the housing 2. The exhaust fans 71 and 72 are provided to suck and discharge air which has been introduced into the housing 2 from the intake holes 251 and 252.

Note that, in each drawing referred to in the following description, XYZ coordinate axes are described such that a direction from the left side plate 25L toward the right side plate 25R is a +X direction, a direction from the rear surface portion 22 toward the front surface portion 21 is a +Y direction, and a direction from the bottom plate 24 toward the top plate 23 is a +Z direction.

In the embodiment, each of the intake hole 251 and the intake hole 252 is constituted by an assembly of a plurality of small holes. In the example illustrated in Fig. 2, a shape of each hole is a shape close to a flat ellipse, but is not limited thereto, and can be set to a circle, a polygon, or the like.

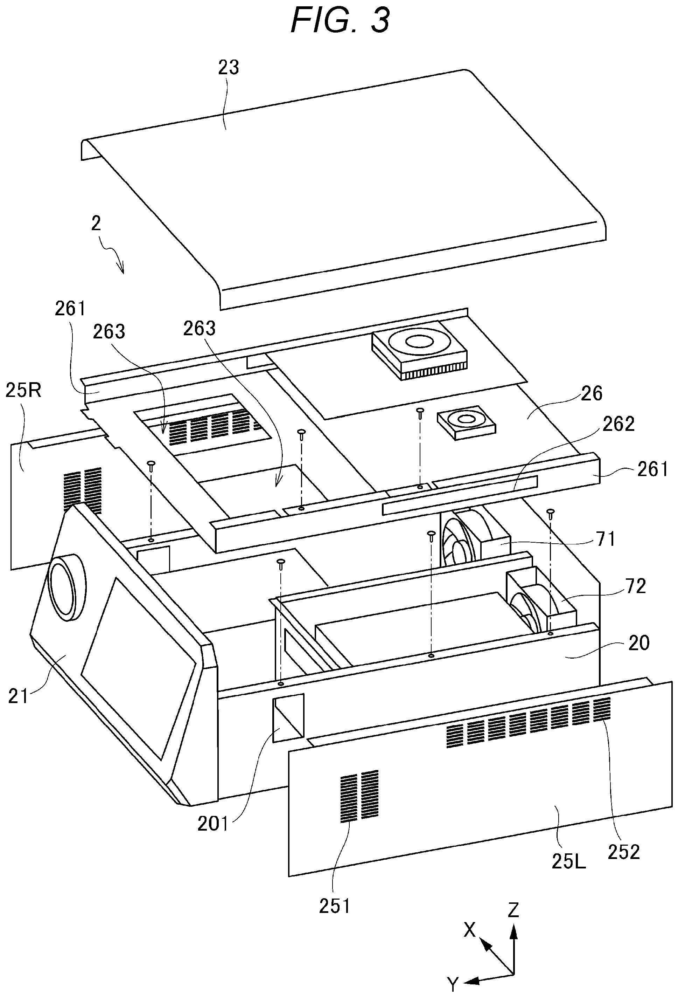

Next, an internal structure of the housing 2 of the electronic endoscope processor 1 will be described with reference to Figs. 3 to 6.

Fig. 3 is a schematic exploded perspective view of the electronic endoscope processor 1 according to the embodiment.

As illustrated in Fig. 3, the housing 2 of the electronic endoscope processor 1 includes a box-shaped frame 20 including the front surface portion 21, the rear surface portion 22, and the bottom plate 24. The left side plate 25L, the right side plate 25R, the top plate 23, and the partition plate 26 are attached to the frame 20.

The partition plate 26 and the top plate 23 are fixed to the frame 20 in this order to partition the internal space of the housing 2 into two spaces of an upper space and a lower space. That is, the housing 2 of the electronic endoscope processor 1 has a two-story structure by the partition plate 26.

In the frame 20, an aperture 201 is formed at a position corresponding to the intake hole 251. Accordingly, when the side plate 25 is attached to the frame 20, outside air can be introduced into the internal space (lower space) of the housing 2 from the intake hole 251 and the aperture 201.

The partition plate 26 has a pair of side wall portions 261 so as to face the left side plate 25L and the right side plate 25R, respectively, and an aperture 262 (an example of a first aperture) is formed in the side wall portion 261. Accordingly, when the side plate 25 is attached to the frame 20, outside air can be introduced into the internal space (upper space) of the housing 2 from the intake hole 252 and the aperture 262.

As illustrated in Fig. 3, two apertures 263 are formed in the partition plate 26. The exhaust fans 71 and 72 are arranged behind the lower space. The aperture 263 is provided to move the air introduced from the intake hole 252 as an air flow from the upper space to the lower space of the housing 2. Note that the number of the apertures 263 is two in the example illustrated in Fig. 3, but is not limited thereto, and the number of the apertures 263 can be appropriately set in order to generate a desired air flow.

In each of the upper space and the lower space of the housing 2, heat generating components such as the circuit boards and the light source device of the electronic endoscope processor 1 are mounted. Therefore, the electronic endoscope processor 1 according to the embodiment is configured to cool these heat generating components in a well-balanced manner.

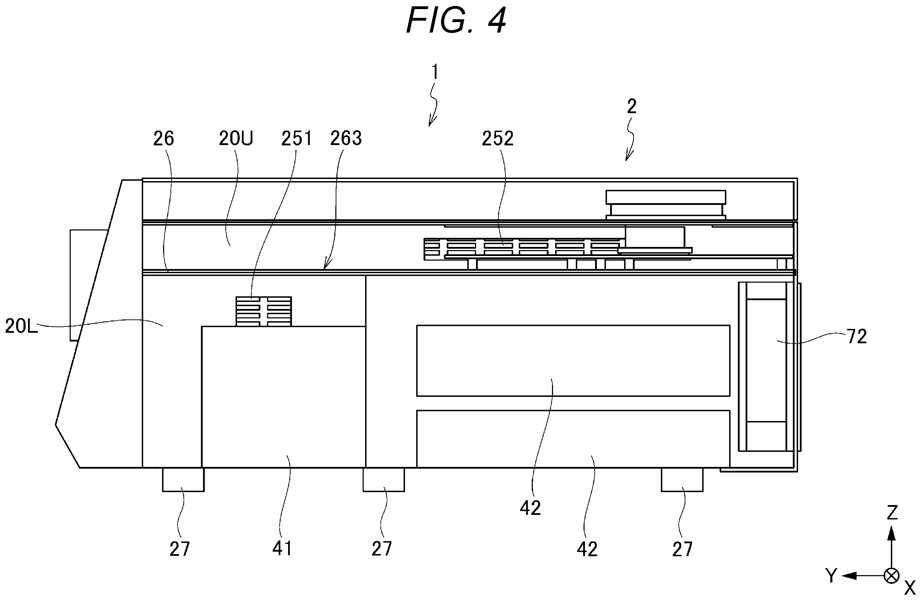

A structure in the housing 2 of the electronic endoscope processor 1 according to the embodiment will be further described with reference to Figs. 4 to 6.

Fig. 4 is a view of the inside of the housing 2 of the electronic endoscope processor 1 according to the embodiment as viewed from the side. As illustrated in Fig. 4, the internal space of the housing 2 is partitioned into an upper space 20U (an example of a second space) and a lower space 20L (an example of a first space) by the partition plate 26. Heat generating components 41 and 42 are arranged in the lower space 20L.

The aperture 263 of the partition plate 26 is preferably arranged in front. In this case, as illustrated in Fig. 4, since the intake hole 252 and the aperture 263 of the partition plate 26 are separated from each other in the front-rear direction, heat generating components mounted in the upper space 20U are uniformly cooled when air at room temperature introduced from the intake hole 252 is directed to the aperture 263.

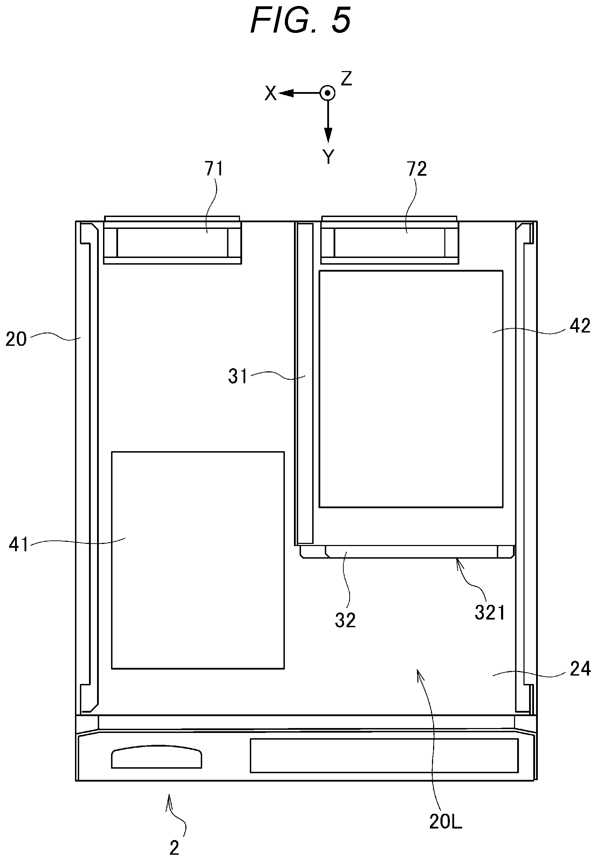

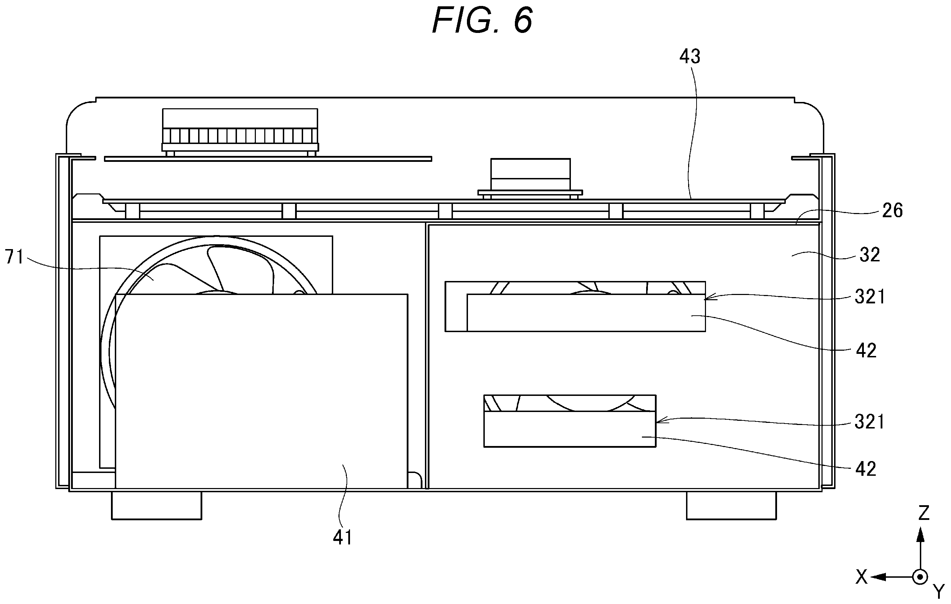

Fig. 5 is a view of the lower space 20L of the housing 2 of the electronic endoscope processor 1 according to the embodiment as viewed from above. Fig. 6 is a view of the inside of the electronic endoscope processor 1 according to the embodiment as viewed from the front.

In the embodiment, an air flow adjusting plate 31 is provided in the lower space 20L of the housing 2. The air flow adjusting plate 31 is a plate standing upward from the bottom plate 24 of the housing 2, and is installed substantially parallel to a direction of an air flow toward the exhaust fans 71 and 72. The air flow adjusting plate 31 is arranged between the aperture 263 and the heat generating component 42 in the lower space 20L, and adjusts an air flow entering the lower space 20L from the aperture 263 to be directed to the heat generating component 42. Since the air flow adjusting plate 31 is provided, the air flow can be divided in accordance with the arrangement of the heat generating components 41 and 42 in the lower space 20L.

In the example illustrated in Fig. 5, the air flow adjusting plate 31 is formed to divide the air flow into two directions toward the respective two exhaust fans 71 and 72.

Note that an air flow adjusting plate for dividing an air flow may be arranged in the upper space 20U as necessary although not illustrated.

A plurality of air flow adjusting plates can also be arranged. For example, in a case where three exhaust fans are provided in the lower space 20L, two air flow adjusting plates may be arranged in parallel in the same direction as the air flow adjusting plate 31 in order to divide the air flow entering the lower space 20L from the upper space 20U toward the respective exhaust fans.

As illustrated in Fig. 5, for example, the air flow adjusting plate 32 may be provided so as to face the direction of the air flow toward the exhaust fan 72.

As illustrated in Fig. 6, the air flow adjusting plate 32 has an aperture 321 (an example of a second aperture) which is an aperture for adjusting a wind speed and/or a wind amount of an air flow directed to each of the heat generating components 42. Although an example in which two apertures 321 are formed is illustrated in the example illustrated in Fig. 6, but the number of the apertures 321 is not limited thereto. The number of the apertures 321 and/or sizes of the apertures 321 can be set such that a wind amount and/or a wind speed corresponding to the heat generating components can be obtained. The number of the apertures 321 is preferably set in accordance with a position of each of the heat generating components 42.

In Fig. 6, a shape of the aperture 321 is rectangular, but is not limited thereto, and can be a desired shape (for example, circular, elliptical, or polygonal) to adjust the air flow.

It is preferable to adjust the size, shape, and position of the aperture 321 to increase the wind speed of the air flow in terms of preventing dust from being accumulated or adhering onto each of the heat generating components 42.

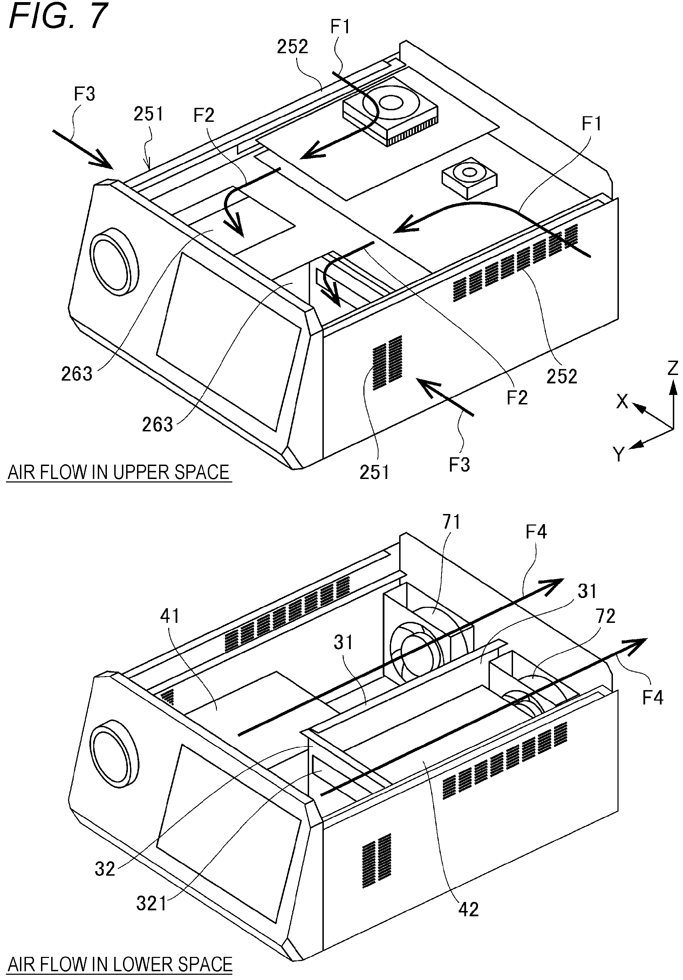

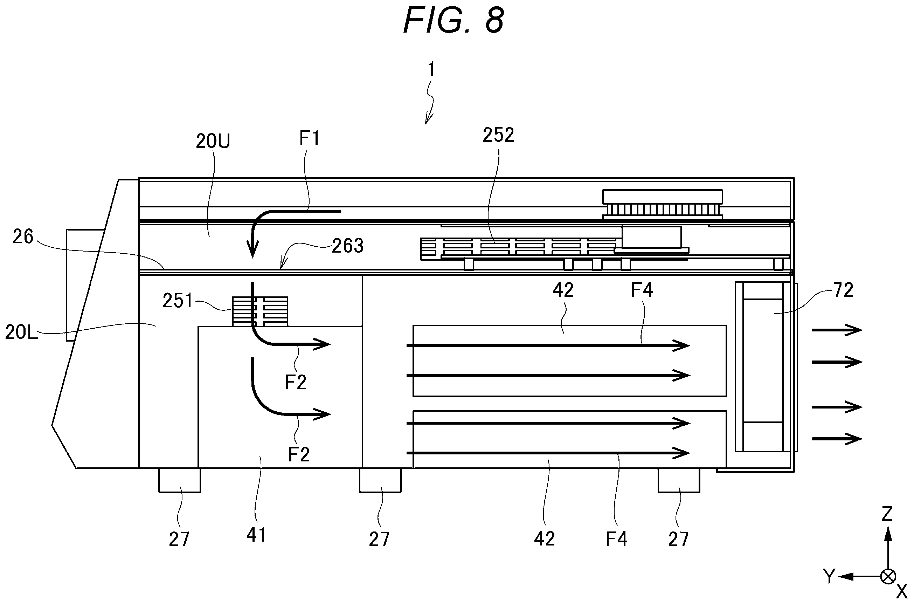

Next, a cooling operation of the electronic endoscope processor 1 will be described with reference to Figs. 7 and 8.

Fig. 7 is a view for describing an air flow in the internal space (upper space and lower space) of the housing 2 of the electronic endoscope processor 1 according to the embodiment. Fig. 8 is a view for describing an air flow when the inside of the housing 2 of the electronic endoscope processor 1 according to the embodiment is viewed from the side.

The air flow in the housing 2 of the electronic endoscope processor 1 is a flow of air in which outside air is introduced into the upper space from the intake hole 252 and exhausted from the exhaust fans 71 and 72 in the lower space.

Referring to Fig. 7, first, air flows F1 introduced from the intake holes 252 into the upper space become air flows F2 directed to the apertures 263 of the partition plate 26 while cooling the heat generating components arranged in the upper space, and are directed to the lower space. Air flows F3 are introduced into the lower space from the intake holes 251. The air flows F2 warmed by heat generated by the heat generating components in the upper space are mixed with the air flows F3 at room temperature, introduced from the intake holes 251, whereby the amount of heat temporarily decreases.

Since the air flow adjusting plates 31 and 32 are provided in the lower space, one of the air flows F2 directed to the lower space from the apertures 263 formed at two locations flows toward the exhaust fan 71 as an air flow F4, and the other of the air flows F2 directed to the lower space flows toward the exhaust fan 72 as an air flow F4. The air flow adjusting plate 32 including the aperture 321 is provided in accordance with the heat generating components 41 and 42 arranged in the lower space, thereby adjusting wind amounts and/or wind speeds of the air flows F4. The size, position, shape, and the like of the aperture 321 of the air flow adjusting plate 32 are adjusted in accordance with the position of the heat generating component 42, thereby locally cooling the heat generating component 42.

As described above, in the housing 2 of the electronic endoscope processor 1 according to the embodiment, since an air flow flows from the intake hole 252 in the upper space toward the exhaust fans 71 and 72 arranged only in the lower space, the air flow does not become non-uniform between the upper space and the lower space, and the internal space of the housing can be cooled in a well-balanced manner. At this time, cooling performance can be adjusted by adjusting the wind amount and/or the wind speed of the air flow by the air flow adjusting plate in accordance with the arrangement of the heat generating components in the housing 2.

In the embodiment, a size of the intake hole 252 is set to be larger than a size of the intake hole 251. Since the intake hole 252 is larger, a lot of outside air can be introduced from the upper space 20U of the housing 2, and a flow rate and/or a flow velocity of the air flow reaching the exhaust fans 71 and 72 can be increased.

In the embodiment, the size of the intake hole 251 is set such that the air flow is the fastest in the lower space 20L and the upper space 20U.

The inventor simulated the flow velocity of the air flow in the internal space of the housing 2 while changing sizes of the intake hole 251 and the intake hole 252. First, when the size of the intake hole 252 through which outside air is introduced into the upper space 20U was reduced from a reference size to 1/2 and 1/3, the flow velocity in the upper space 20U decreased in both cases, and thus it has been confirmed that it is preferable to increase the size of the intake hole 252. Furthermore, when the size of the intake hole 251 through which outside air is introduced into the lower space 20L was set to (reduced to) 1/2 or (enlarged to) 3/2, the flow velocity of the air flow increased in both the upper space 20U and the lower space 20L when the size of the intake hole 251 was reduced, and the flow velocity of the air flow decreased in both the upper space 20U and the lower space 20L when the size of the intake hole 251 was increased.

From the above simulation results, it has been confirmed that the air flow can be set to be the fastest in the lower space 20L and the upper space 20U by adjusting the size of the intake hole 251.

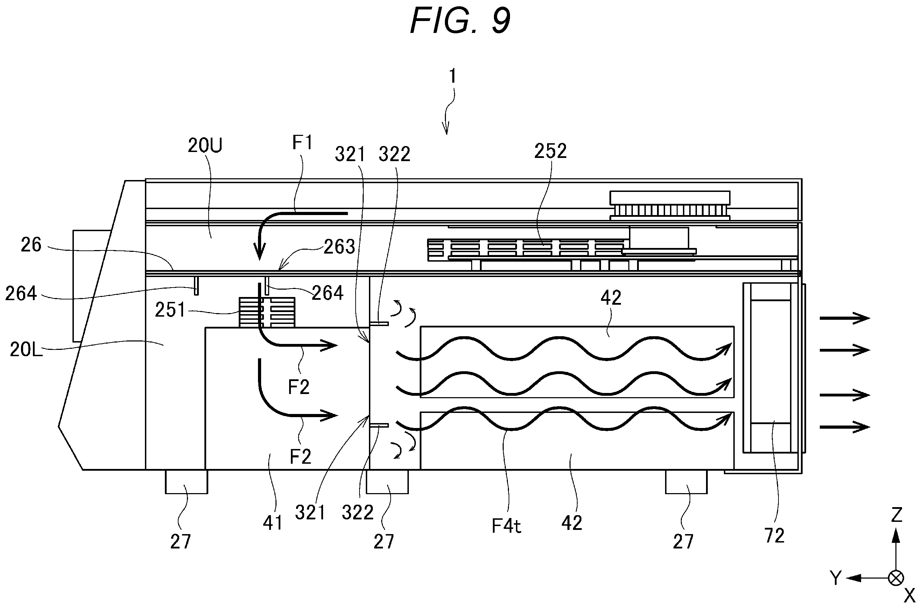

In the embodiment, a turbulence generating structure that generates turbulence in the air flow passing through the aperture 263 of the partition plate 26 and/or the aperture 321 of the air flow adjusting plate 32 may be provided. For example, Fig. 9 illustrates an example in which protrusions 264 and 322, which serve as the turbulence generating structure, are provided on peripheral edges of the aperture 263 and the aperture 321, respectively. Note that, as the turbulence generating structure, bending or a flap can be adopted in addition to the protrusions.

The electronic endoscope processor of the present invention has been described above in detail, but the electronic endoscope processor of the present invention is not limited to the foregoing embodiment, and may of course be modified or altered in various ways in a range not deviating from the scope and spirit of the present invention.

The present invention relates to a patent application of Japanese Patent Application No. 2022-120374 filed with the Japan Patent Office on July 28, 2022, the entire contents of which are incorporated herein by reference.

## Claims

1. An electronic endoscope processor comprising:
a housing;
a partition plate that partitions an internal space of the housing into a first space below and a second space above, the partition plate having a first aperture that is an aperture allowing the first space and the second space to communicate with each other; and
an exhaust fan that is attached to the housing and exhausts air in the first space to an outside,
wherein a first intake hole through which outside air is acquired into the first space and a second intake hole through which outside air is acquired into the second space are formed in the housing.

2. The electronic endoscope processor according to claim **1,** further comprising:
one or a plurality of heat generating components that are arranged in the first space and generate heat during operation; and
an air flow adjusting plate that is arranged between the first aperture and each of the heat generating components in the first space and adjusts an air flow entering the first space through the first aperture to be directed to each of the heat generating components.

3. The electronic endoscope processor according to claim 2, wherein
a second aperture that is an aperture for adjusting a wind speed and/or a wind amount of the air flow directed to each of the heat generating components is formed in the air flow adjusting plate.

4. The electronic endoscope processor according to any one of claims 1 to 3, wherein
the second intake hole is larger than the first intake hole.

5. The electronic endoscope processor according to any one of claims 1 to 3, wherein
a size of the first intake hole is set in such a manner that an air flow is fastest in the first space and the second space.

6. The electronic endoscope processor according to any one of claims 1 to 3, wherein
a peripheral edge of the first aperture has a turbulence generating structure that generates turbulence in an air flow passing through the first aperture.

7. The electronic endoscope processor according to claim 3, wherein
a peripheral edge of the second aperture has a turbulence generating structure that generates turbulence in an air flow passing through the second aperture.
